# EUROPEAN PATENT SPECIFICATION

(11) **EP 1 377 667 B1**
(45) Date of publication and mention of the grant of the patent: **19.11.2008**
(21) Application number: 02714638.0
(22) Date of filing: 28.03.2002
(51) Int. Cl.: C12N 15/62, C07K 14/01, C12N 9/12, A61K 47/48, A61K 38/16, A61K 38/45, A61P 35/00, A61P 19/02

(54) **FUSION PROTEINS FOR SPECIFIC TREATMENT OF CANCER AND AUTO-IMMUNE DISEASES**
FUSIONSPROTEINE ZUR SPEZIFISICHEN BEHANDLUNG VON KREBS UND AUTOIMMUNKRANKHEITEN
PROTEINES DE FUSION POUR LE TRAITEMENT SPECIFIQUE DU CANCER ET DE MALADIES AUTO-IMMUNES

(30) Priority: 30.03.2001 US 280229 P
(43) Date of publication of application: 07.01.2004
(73) Proprietor: Leadd B.V., 2333 AL Leiden (NL)
(72) Inventor: NOTEBORN, Mathieu, Hubertus, Maria, NL-2352 EH Leiderdorp (NL); RENES, Johan, NL-3766 WL Soest (NL); ZHANG, Ying-Hui, NL-2334 ET Leiden (NL)
(74) Representative: Prins, Adrianus Willem
(86) International application number: PCT/NL2002/000204
(87) International publication number: WO 2002/079222

(56) References cited:
- EP-A- 1 186 665
- EP-A- 1 188 832
- WO-A-00/41497
- WO-A-96/41191
- WO-A-98/46760
- WO-A-99/08108
- WO-A-02/085305
- DATABASE BIOSIS [Online] BIOSCIENCES INFORMATION SERVICE, PHILADELPHIA, PA, US; April 1998 (1998-04) VAN DER EB M M ET AL: "Severe hepatic dysfunction after adenovirus-mediated transfer of the herpes simplex virus thymidine kinase gene and ganciclovir administration." Database accession no. PREV199800256147 XP002229399 cited in the application & GENE THERAPY, vol. 5, no. 4, April 1998 (1998-04), pages 451-458, ISSN: 0969-7128
- DATABASE BIOSIS [Online] BIOSCIENCES INFORMATION SERVICE, PHILADELPHIA, PA, US; 1 May 1999 (1999-05-01) GOOSSENS PAULIEN H ET AL: "Feasibility of adenovirus-mediated nonsurgical synovectomy in collagen-induced arthritis-affected rhesus monkeys." Database accession no. PREV199900277375 XP002229400 & HUMAN GENE THERAPY, vol. 10, no. 7, 1 May 1999 (1999-05-01), pages 1139-1149, ISSN: 1043-0342
- DATABASE BIOSIS [Online] BIOSCIENCES INFORMATION SERVICE, PHILADELPHIA, PA, US; 1989 LAFYATIS R ET AL: "ANCHORAGE-INDEPENDENT GROWTH OF SYNOVIOCYTES FROM ARTHRITIC AND NORMAL JOINTS STIMULATION BY EXOGENOUS PLATELET-DERIVED GROWTH FACTOR AND INHIBITION BY TRANSFORMING GROWTH FACTOR-BETA AND RETINOIDS" Database accession no. PREV198987122407 XP002229401 cited in the application & JOURNAL OF CLINICAL INVESTIGATION, vol. 83, no. 4, 1989, pages 1267-1276, ISSN: 0021-9738

### SEQUENCE LISTING

<110> Noteborn, Mathieu H.M.
   Renes, Johan
   zhang, Ying-Hui
<120> Fusion proteins for specific treatment of cancer and auto-immune diseases
<130> P55985PC00
<140> PCT/NL02/00204
   <141> 2002-03-28
<150> us 10/113,790
   <151> 2002-03-29
<150> US 60/280,229
   <151> 2001-03-30
<160> 6
<170> PatentIn version 3.1
<210> 1
   <211> 523
   <212> PRT
   <213> Artificial Sequence
<220>
   <223> Apoptin-TK fusion protein
<400> 1
   Met Asn Ala Leu Gln Glu Asp Thr Pro Pro Gly Pro Ser Thr Val Phe
   1               5                   10                  15
   Arg Pro Pro Thr Ser Ser Arg Pro Leu Glu Thr Pro His Cys Arg Glu
               20                  25                  30
   Ile Arg Ile Gly Ile Ala Gly Ile Thr Ile Thr Leu Ser Leu Cys Gly
   35 40 45
   Cys Ala Asn Ala Arg Ala Pro Thr Leu Arg Ser Ala Thr Ala Asp Asn
   50 55 60
   Ser Glu Ser Thr Gly Phe Lys Asn Val Pro Asp Leu Arg Thr Asp Gln
   65 70 75 80
   Ala Lys Pro Pro Ser Lys Lys Arg Ser Cys Asp Pro Ser Glu Tyr Arg
   85 90 95
   Val Ser Glu Leu Lys Glu Ser Leu Ile Thr Thr Thr Pro Ser Arg Pro
   100 105 110
   Arg Thr Ala Lys Arg Arg Ile Arg Leu Ala Gly Glu Gln Arg Ala Ala
   115 120 125
   Asp Leu Gly Gly Val Lys Leu His Leu Pro Phe Gly Gln Arg Leu Val
   130 135 140
   Glu Ala Arg Ala Ser Tyr Pro Cys His Gln His Ala Ser Ala Phe Asp
   145 150 155 160
   Gln Ala Ala Arg Ser Arg Gly His Asn Asn Arg Arg Thr Ala Leu Arg
   165 170 175
   Pro Arg Arg Gln Gln Lys Ala Thr Glu Val Arg Leu Glu Gln Lys Met
   180 185 190
   Pro Thr Leu Leu Arg Val Tyr Ile Asp Gly Pro His Gly Met Gly Lys
   195 200 205
   Thr Thr Thr Thr Gln Leu Leu Val Ala Leu Gly Ser Arg Asp Asp Ile
   210 215 220
   Val Tyr Val Pro Glu Pro Met Thr Tyr Trp Arg Val Leu Gly Ala Ser
   225 230 235 240
   Glu Thr Ile Ala Asn Ile Tyr Thr Thr Gln His Arg Leu Asp Gln Gly
   245 250 255
   Glu Ile Ser Ala Gly Asp Ala Ala Val Val Met Thr Ser Ala Gln Ile
   260 265 270
   Thr Met Gly Met Pro Tyr Ala Val Thr Asp Ala Val Leu Ala Pro His
   275 280 285
   Ile Gly Gly Glu Ala Gly Ser Ser His Ala Pro Pro Pro Ala Leu Thr
   290 295 300
   Leu Ile Phe Asp Arg His Pro Ile Ala Ala Leu Leu Cys Tyr Pro Ala
   305 310 315 320
   Ala Arg Tyr Leu Met Gly Ser Met Thr Pro Gln Ala Val Leu Ala Phe
   325 330 335
   Val Ala Leu Ile Pro Pro Thr Leu Pro Gly Thr Asn Ile Val Leu Gly
   340 345 350
   Ala Leu Pro Glu Asp Arg His Ile Asp Arg Leu Ala Lys Arg Gln Arg
   355 360 365
   Pro Gly Glu Arg Leu Asp Leu Ala Met Leu Ala Ala Ile Arg Arg Val
   370 375 380
   Tyr Gly Leu Leu Ala Asn Thr Val Arg Tyr Leu Gln Gly Gly Gly Ser
   385 390 395 400
   Trp Arg Glu Asp Trp Gly Gln Leu Ser Gly Ala Ala Val Pro Pro Gln
   405 410 415
   Gly Ala Glu Pro Gln Ser Asn Ala Gly Pro Arg Pro His Ile Gly Asp
   420 425 430
   Thr Leu Phe Thr Leu Phe Arg Ala Pro Glu Leu Leu Ala Pro Asn Gly
   435 440 445
   Asp Leu Tyr Asn Val Phe Ala Trp Ala Leu Asp Val Leu Ala Lys Arg
   450 455 460
   Leu Arg Pro Met His Val Phe Ile Leu Asp Tyr Asp Gln Ser Pro Ala
   465 470 475 480
   Gly Cys Arg Asp Ala Leu Leu Gln Leu Thr Ser Gly Met Val Gln Thr
   485 490 495
   His Val Thr Thr Pro Gly Ser Ile Pro Thr Ile Cys Asp Leu Ala Arg
   500 505 510
   Thr Phe Ala Arg Glu Met Gly Glu Ala Asn Glx
   515 520
<210> 2
   <211> 1594
   <212> DNA
   <213> Artificial sequence
<220>
   <223> Encoding sequence for Apoptin-TK fusion protein
<400> 2
   ggatccgtcg accatgaacg ctctccaaga agatactcca cccggaccat caacggtgtt      60
   caggccacca acaagttcac ggccgttgga aacccctcac tgcagagaga tccggattgg     120
   tatcgctgga attacaatca ctctatcgct gtgtggctgc gcgaatgctc gcgctcccac     180
   gctaagatct gcaactgcgg acaattcaga aagcactggt ttcaagaatg tgccggactt     240
   gaggaccgat caacccaagc ctccctcgaa gaagcgatcc tgcgacccct ccgagtacag     300
   ggtaagcgag ctaaaagaaa gcttgattac cactactccc agccgacccc gaaccgcaaa     360
   aaggcgtata agactggcag gagaacagcg tgccgcagat cttggtggcg tgaaactcca     420
   cctcccgttc ggccagcgcc ttgtagaagc gcgtgcttcg tacccctgcc atcaacacgc     480
   gtctgcgttc gaccaggctg cgcgttctcg cggccataac aaccgacgta cggcgttgcg     540
   ccctcgccgg cagcaagaag ccacggaagt ccgcctggag cagaaaatgc ccacgctact     600
   gcgggtttat atagacggtc ctcacgggat ggggaaaacc accaccacgc aactgctggt     660
   ggccctgggt tcgcgcgacg atatcgtcta cgtacccgag ccgatgactt actggcaggt     720
   gctgggggct tccgagacaa tcgcgaacat ctacaccaca caacaccgcc tcgaccaggg     780
   tgagatatcg gccggggatg cggcggtggt aatgacaagc gcccagataa caatgggcat     840
   gccttatgcc gtaaccgacg ccgttctggc tcctcatatc gggggggagg ctgggagctc     900
   acatgccccg cccccggccc tcaccctcat cttcgaccgc catcccatcg ccgccctcct     960
   gtgctacccg gccgcgcgat accttatggg cagcatgacc ccccaggccg tgctggcgtt    1020
   cttggccctc atcccgccga ccttgcccgg cacaaacatc gtgttggggg cccttccgga    1080
   ggacagacac atcgaccgcc tggccaaacg ccagcgcccc ggcgagcggc ttgacctggc    1140
   tatgctggcc gcgattcgcc gcgtttacgg gctgcttgcc aatacggtgc ggtatctgca    1200
   gggcggcggg tcgtggcggg aggattgggg acagctttcg gggacggccg tgccgcccca    1260
   gggtgccgag ccccagagca acgcgggccc acgaccccat atcggggaca cgttatttac    1320
   cctgtttcgg gcccccgagt tgctggcccc caacggcgac ctgtataacg tgtttgcctg    1380
   ggccttggac gtcttggcca aacgcctccg tcccatgcac gtctttatcc tggattacga    1440
   ccaatcgccc gccggctgcc gggacgccct gctgcaactt acctccggga tggtccagac    1500
   ccacgtcacc accccaggct ccataccgac gatctgcgac ctggcgcgca cgtttgcccg    1560
   ggagatgggg gaggctaact gagaattcgg atcc                                1594
<210> 3
   <211> 34
   <212> DNA
   <213> Artificial Sequence
<220>
   <223> primer 1
<400> 3
   cgggatccgt cgaccatgaa cgctctccaa gaag 34
<210> 4
   <211> 65
   <212> DNA
   <213> Artificial sequence
<220>
   <223> primer 2
<400> 4
   gtggagtttc acgccaccaa gatctgcggc acgctgttct cctgccagtc ttatacgcct 60
   ttttg 65
<210> 5
   <211> 67
   <212> DNA
   <213> Artificial Sequence
<220>
   <223> primer 3
<400> 5
   gtgaaactcc acctcccgtt cggccagcgc cttgtagaag cgcgtgcttc gtacccctgc 60
   catcaac 67
<210> 6
   <211> 33
   <212> DNA
   <213> Artificial sequence
<220>
   <223> primer 4
<400> 6
   cgggatccga attctcagtt agcctccccc atc 33

## Description

Field: The present invention relates to the field of therapies based upon molecular biology. The invention further relates to the field of cancer and/or auto-immune diseases. Where in this specification reference is made to either, the other should be included unless expressly excluded. The invention also relates to induction of apoptosis in cells associated with cancer or to auto-immune diseases.

Background: Apoptosis is an active and programmed physiological process for eliminating superfluous, altered or malignant cells (Earnshaw, 1995, Duke et al., 1996). Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighbouring cells and/or macrophages will rapidly phagocytose these dying cells (Wyllie et al., 1980, White, 1996). The apoptotic process can be initiated by a variety of regulatory stimuli (Wyllie, 1995, White 1996, Levine, 1997).

Changes in the cell survival rate play an important role in human pathogenesis, for example, in cancer development and autoimmune diseases, which is caused by enhanced proliferation but also by decreased cell death (Kerr et al., 1994, Paulovich, 1997). A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).

Many tumors, however, acquire a mutation in p53 during their development, often correlating with poor response to cancer therapy. Certain transforming genes of tumorigenic DNA viruses can inactivate p53 by directly binding to it. Examples of such agents are the E6 protein from oncogenic subtypes of the Human Papiloma Virus and the large T antigen of the tumor DNA virus SV40 it (Teodoro, 1997). Another example of the emergence of a strong resistance to various apoptosis-inducing hemotherapeutic agents in (leukemic) tumors is the association of a high expression level of the proto-oncogene Bcl-2 or Bcr-abl with reduced sensitivity of these tumors to therapy (Hockenberry 1994, Sachs and Lotem, 1997).

Autoimmune diseases are a group of severe diseases, which are characterized by inflammatory disorders, such as Crohn's disease and rheumatoid arthritis (RA). Recently, evidence has been provided that RA-related fibroblast-like synoviocytes exhibit characteristics of transformed/tumorigenic cells. For instance, adherence to plastic or extracellular matrix is generally required for normal fibroblasts to proliferate and survive in culture for prolonged periods of time. Transformed cells, however, can grow in suspension in semi-solid medium without contact with a solid surface. While FLS typically grow and thrive under conditions that permit adherence, they can, in some circumstances, proliferate in an anchorage-independent manner (Lafyatis et al., 1989). Furthermore, the expression of several oncogenes such as c-myc has been reported for cultured FLS (Gay and Gay, 1989). Higher endogenous release of growth factors such as tumor growth factor-beta and other cytokines have also been described for FLS (Bucala et al., 1991; Remmers et al., 1990; Geiler, 1994; Firestein, 1995 and 1995a). Also, in some cases non-functional tumor-suppressor p53 has been related with RA (Aupperle et al., 1998). Although mutant p53 is not an oncogene, it prevents induction of apoptosis by endogenous or exogenous agents. All these data indicate that FLS are irreversibly altered in RA and that an autonomous process allows them to remain activated even after removal from the articular inflammatory milieu (Firestein, 1995).

Therefore, therapies for cancer and auto-immune diseases such as RA need to be developed that can restore the cell death process in the disease-related cells. Among the broad array of genes that have been evaluated for tumor therapy, those encoding prodrug activation enzymes are especially appealing as they directly complement ongoing clinical chemotherapeutic regimes. These enzymes can activate prodrugs that have a low inherent toxicity using both bacterial and yeast enzymes, or enhance prodrug activation by mammalian enzymes. Activation of ganciclovir by viral thymidine kinase is currently being evaluated in clinical trials.

In, for example, cancer gene therapy, vectors target delivery of therapeutic genes to tumor cells by means of direct injection into the tumor mass or surrounding tissues. The activated drug is able to act on non-transduced tumor cells. This "by-stander effect" can even act at distant sites and is believed to be mediated by the immune system (Aghi et al., 2000). However, several drawbacks of this approach have been reported. For example, Van der Eb et al. (1998) have reported that treatment of rats with an adenovirus synthesizing Herpes Simplex Virus thymidine kinase (HSV TK) and ganciclovir administration resulted in a severe hepatic dysfunction. These studies clearly showed that normal (non-transformed) non-mitotic tissues can be affected by adenovirus mediated HSV TK transfer and subsequent ganciclovir treatment. Given the hepatropic nature of systemically nature of systemically administered adenovirus-derived vectors, it will be essential to monitor liver functions of patients included in all gene therapy trials involving adenoviral vectors with the HSV-TK gene. Furthermore, over expression of the proto-oncogene Bcl-2, which has anti-apoptotic activity, significantly increased tumor cell resistance against a large panel of cytotoxic drugs and also against HSV-TK/ganciclovir mediated gene therapy (Fels et al., 2000). Therefore, one needs approaches, which will make the HSV-TK approach based on adenoviral gene therapy less toxic. Furthermore, strategies have to be developed circumventing the resistance against activated prodrugs.

Apoptin (also called "vp3"; the terms may be used interchangeable herein) is a small protein derived from chicken anemia virus ("CAV"; Noteborn and De Boer, 1996, Noteborn et al., 1991, Noteborn et al., 1994; 1998a) which induces apoptosis in human malignant and transformed cell lines. *In vitro* and *in vivo,* apoptin fails to induce programmed cell death in normal lymphoid, dermal, epidermal, endothelial and smooth-muscle cells. However, when normal cells are transformed they become susceptible to apoptosis induced by apoptin. In normal cells, apoptin was found predominantly in the cytoplasm, whereas in transformed or malignant cells i.e. characterized by hyperplasia, metaplasia or dysplasia, it was located in the nucleus (Danen-van Oorschot, 1997 and Noteborn, 1996).

Long-term expression of apoptin in normal human fibroblasts revealed that apoptin apparently has no toxic or transforming activity in these cells (Danen-van Oorschot, 1997 and Noteborn, 1996). Noteborn and Pietersen (1998) and Pietersen et al. (1999) have provided evidence that adenovirus expressed apoptin does not have an acute toxic effect *in vivo,* whereas in nude mice it has a strong anti-tumor activity. Further evidence of a lack of toxicity *in vivo* comes from transgenic mice which express apoptin from an MHC-I promoter and which have no observable abnormalities (Noteborn and Zhang, 1998). Of importance in the treatment of tumors that have become resistant to chemo or radiation therapy is that apoptin-induced apoptosis occurs in the absence of functional p53 (Zhuang et al., 1995a), and cannot be blocked by Bcl-2, Bcr-abl (Zhuang et al., 1995), or the Bcl-2-associating protein BAG-1 (Danen-Van Oorschot, 1997a, Noteborn,1996). In addition, it appears, that even pre-malignant, minimally transformed cells, may be sensitive to the death-inducing effect of apoptin (Noteborn and Zhang, 1998). Recently, Noteborn and Pietersen (2000) provided evidence that apoptin can recognize the transformed-like autoimmune conditions (e.g. RA), which results in apoptin-induced apoptosis in RA-affected fibroblast-like synoviocytes.

### DISCLOSURE OF THE INVENTION

To further enlarge the array of therapeutic anti-cancer or anti-auto-immune-disease compounds available in the art, additional therapeutic tools are desired. The invention provides novel therapeutic substances, for example novel therapeutic proteinaceous compounds that can contain apoptin jointly with other proteinaceous cytotoxic protein or protein fragments such as HSV TK, or (non) viral vector systems expressing these fusion proteins, especially in those cases when cells are derailed such as cancer-derived or auto-immune-derived cells. In particular the invention describes compounds for use in a therapy based on the activation of cytotoxic compounds and/or making cytotoxic compounds more specific for tumor cells and cells related to autoimmune diseases by binding it to apoptin protein.

In a first embodiment, the invention provides a fusion protein consisting of HSV TK and apoptin that induces apoptosis in a tumor-specific way. The TK-apoptin fusion protein exerts its tumor-specific cytotoxicity when administered to cells.

The invention includes a gene delivery vehicle (or vector) which enables using the features of the tumor-specific apoptin and an enzyme that can activate cytotoxic prodrugs, for cancer and autoimmune disease treatment via the use of gene therapy. Such a gene delivery vehicle, which is an independently infectious vector can for example be a virus, or a liposome, or a polymer, or the like, that in itself can infect or in any other way deliver genetic information to for example tumor cells that can be treated. The genetic information comprises a nucleic acid molecule encoding apoptin-TK-like activity.

Additionally, the invention includes a gene delivery system that in itself is a replication-defective virus but which can replicate in helper or packaging cells to generate progeny gene delivery vehicles. The gene delivery vehicle thus provided by the invention can, for instance, be an adenovirus, parvovirus, or a retrovirus or other DNA or RNA recombinant viruses that can be used as delivery vehicle or a plasmovirus.

Additionally, the invention provides a gene delivery vehicle which has additionally been supplemented with a specific ligand or target molecule or target molecules, by which the gene delivery vehicle can be specifically directed to deliver its genetic information at a target cell of choice. Such a target molecule, or antibody, is reactive with a tumor cell surface receptor or protein.

The invention furthermore includes all steps needed for the construction of a recombinant, replication-defective adenovirus expressing the TK-apoptin fusion product. High titers of recombinant-TK-apoptin adenovirus can be produced by means of adenovirus packaging cell lines, such as 293, 911 and PER.C6. (Noteborn and Pietersen, 1998.) The TK-apoptin does not exhibit a detectable negative effect on all necessary adenovirus replication steps and other adenovirus life-cycle events under cell culture conditions.

Recombinant replication-defective adenovirus expresses TK-apoptin in high amounts in various tumor and/or cells related to auto-immune diseases resulting in the induction of apoptosis. In contrast, expression of TK-apoptin -with or without ganciclovir treatment- in normal non-transformed human cells by means of recombinant adenovirus does not result in the induction of TK-apoptin induced cell death.

In particular, the invention relates to the use of the compounds of the invention in the manafacture of medicaments for use in anti-tumor and anti-autoimmune-disease therapies. Treatment of tumor cells and/or cells related to autoimmune diseases will take place by expression of TK-apoptin fusion protein by means of infecting cells with gene delivery vehicles such as adenovirus vectors that contain a coding sequence for a protein with TK-apoptin-like activity. Therefore, the invention provides gene delivery vehicles such as the adenovirus expressing apoptin, which is a very potential anti-tumor or anti-autoimmune-disease agent. Adenovirus regulation ofTK-apoptin after ganciclovir treatment does not detectably induce apoptosis in human normal non-transformed cells, indicating that the toxicity of *in vivo* treatment with recombinant TK-apoptin adenovirus will be low.

Expression of TK-apoptin in tumor/autoimmune-disease-related cells may also take place by infecting cells with other DNA and/or RNA viral vectors, besides adenovirus vectors, that contain a coding sequence for TK-apoptin. In addition, virus-derived vector systems, such as plasmoviruses can be used for the induction of TK-apoptin induced apoptosis in tumor cells.

Moreover, TK-apoptin fusion protein or derivatives of it will also be effective against tumors which have become resistant to (chemo)-therapeutic induction of apoptosis, due to the lack of functional p53 and/or (over)-expression of Bcl-2 and other apoptosis-inhibiting agents.

To eradicate a tumor, it is imperative that all cells of the tumor, including its potential mini-metastases, are removed upon treatment. Due to the by-stander effect observed by infection of human tumor cells with recombinant adenovirus synthesizing TK-apoptin protein, *in vivo* tumor cells undergo cell death despite the fact that they were not transduced and able to produce themselves TK-apoptin protein. This will result in the complete regression of the treated tumor and most likely also of distal tumors too.

The invention also describes that the activity and behavior of the recombinant proteins is similar to the activity of TK-apoptin protein produced by transcription and translation of the TK-apoptin DNA. In particular, the detailed description provides evidence that micro-injection of TK-apoptin fusion protein, produced in *E. coli* cells and purified, result in induction of apoptosis in human tumor and/or RA-derived cells but not in normal human primary cells.

The invention also has the ability to differentiate between normal and transformed cells that recombinant TK-apoptin protein harbors potential activity for the destruction of tumor cells, or other hyperplasia, metaplasia or dysplasia, with minimal or no toxicity to normal tissue. This invention also describes another example of an effective anti-tumor therapy based on apoptin-derived proteinaceous substances.

Direct introduction of TK-apoptin protein into cells can be achieved *in vitro* and *in vivo* by coupling this effector protein (henceforth referred to as the cargo) to a protein transduction domain. The first description of the capability of certain proteins to cross cell membranes was given independently by Green and Loewenstein (1988) and Frankel and Pabo (1988), for the HIV TAT protein. Henceforth it was shown that synthetic peptides containing the amino acids 48 to 60 derived from the HIV TAT protein could transduce into cells (Vives et al., 1997). This ability can be conferred in trans by chemical cross-linking the TAT-derived protein transduction domain to the cargo protein, enabling proteins as large as 120 kDa to be delivered intra-cellularly, *in vitro* as well as *in vivo* (Fawell et al., 1994). Other transduction domains have been described in the Antennapedia protein from Drosophila melanogaster (Derossi et al., 1998), and several synthetic peptides (Lindgren et al., 2000). The HIV-TAT-mediated process does not depend on endocytosis and is not mediated through a cellular receptor. This explains the remarkable universality that is seen; the proteins can be transduced into all cells tested thus far (Schwarze and Dowdy, 2000). An efficient method based on the HIV TAT peptide has been developed to make recombinant proteins that can be transduced both in vitro and in vivo.

Significantly, when administered *in vivo,* all tissues, including the brain, can be targeted with a recombinant protein (Schwarze, 1999) using this system. Another class of delivery method is based on the hydrophobic core region of signal peptides (Hawiger, 1999). The cellular import of fusions or conjugations between these transduction domains and a cargo are concentration and temperature sensitive. It is, however, cell type independent and a whole range of cells have been shown to be susceptible to internalization of cargo mediated by this class of transduction domains.

In general for the transduction protein technology the drawback is that it has not yet been possible to target a specific (tumor) cell compartment with this system. The lack of tumor specific targeting has thus far prevented the development of an efficient anti-tumor therapy, which uses protein transduction.

In a preferred embodiment, the present invention describes compounds, which enable circumvention of this drawback. Transduced cells, which take up TK-apoptin-derived protein are only undergoing cell death when they are of a transformed or malignant nature and stay alive when they are normal. This means that the use ofTK-apoptin as part of a transduction-capable proteinaceous substance will make it a potential anti-tumor agent.

The invention describes another way to introduce (recombinant)TK-apoptin fusion protein into cells, which is by fusion of the protein with a ligand. Receptor mediated internalization then results in the uptake of the fusion protein. An example for such apoptin fusion proteins are based on the Epidermal Growth Factor ("EGF"). All these methods hinge on the activity of the recombinant or purified cargo-protein in the target cell.

In particular, the invention shows that TK-apoptin protein produced in various ways retains its specific tumor killing ability, and thus opens the way to combine the generalized delivery of protein transduction with the specific anti-tumor activity of apoptin and with the by-stander effect of HSV-TK-ganciclovir, which results in a new method by which transformed or malignant cells can be eradicated. Thus, the invention describes several examples of TK-apoptin-derived proteinaceous substances cross-linked to a transduction domain such as TAT can be applied.

Furthermore, the invention describes other transduction domains as mentioned above can be envisaged, where all share the capacity to introduce the TK-apoptin protein into tumor cells and normal cells alike. Fusion to a ligand may specifically target TK-apoptin to one cell type, but the tumor specificity of TK-apoptin will be pivotal for therapeutic applications with minimal collateral damage to normal cells. As an example, EGF-targeted TK-apoptin could be introduced into all EGF-receptor expressing cells. TK-Apoptin will, however, destroy only the tumor cells. In addition, due to the by-stander effect of TK-apoptin also tumor cells that did not contain the TK-apoptin fusion protein will undergo cell death.

The invention provides the application of cell-permeable protein as a drug being much more safe in the long term than gene-therapy approaches possibly causing genetic alterations resulting in diseases such as cancer.

The invention will be explained in more detail in the following description, which is not limiting the invention.

### DESCRIPTION OF THE FIGURES

FIG. 1. Schematic representation of Apoptin-TK fusion
FIG. 2. Amino acid sequence of the TK-apoptin fusion protein
FIG. 3. DNA sequence of the TK-apoptin fusion protein

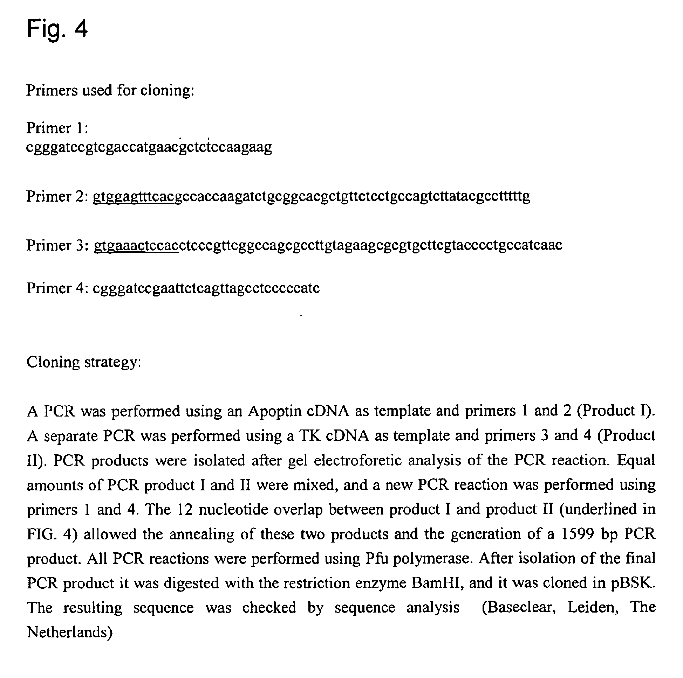

FIG. 4. Description of the cloning strategy and the used linkers for resulting in the final TK-apoptin constructs.

### DETAILED DESCRIPTION OF THE INVENTION

The invention includes a fusion protein comprising a polypeptide providing cytotoxicity, the polypeptide being fused to a moiety, namely apoptin, rendering the fusion protein functionally available in aberrant cells and not functionally available in non-aberrant cells. The polypeptide provides an enzymatic activity that will convert a prodrug into a drug.

In a preferred embodiment, the polypeptide is TK and the moiety is apoptin.

The fusion protein may be conjugated to a targeting molecule for preferential delivery to cancer cells. For instance, the targeting molecule may be a liposome, folic acid, a folic acid derivative, vitamin B-12, vitamin B-12 derivative, an antibody, antibody fragment, or a ligand for a receptor.

In a preferred in vitro method according to the invention, such includes administering a fusion protein having a moiety rendering the fusion protein functionally available in aberrant cells and not functionally available in non-aberrant cells, said moiety being apoptin.

In another embodiment, the invention includes a gene delivery vehicle encoding a fusion protein comprising the moiety rendering the fusion protein functionally available in aberrant cells and not functionally available in non-aberrant cells. For instance, the gene encoding the fusion protein can encode a targeting polypeptide. Also, the targeting polypeptide comprises a transduction domain such as TAT.

The targeting polypeptide can also comprise a member of a specific binding pair or a scFv.

The invention also includes an in vitro method for providing aberrant cells predominantly over normal cells with a desired fusion protein comprising administering a fusion protein having the moiety rendering the fusion protein functionally available in aberrant cells and not functionally available in non-aberrant cells.

Delivery vehicles can comprise adenovirus, retro, AAV, plasmovirus, nonviral polyphosphosines, lyposomes, etc.

The invention furthermore provides or describes all steps needed for the construction of a recombinant, replication-defective adenovirus expressing the TK-apoptin fusion product. High titers of recombinant-TK-apoptin adenovirus can be produced by means of adenovirus packaging cell lines, such as 293, 911 and PER.C6. The fact that recombinant-TK-apoptin adenovirus vectors can become produced by the herein described system, implies that (conditional) replicative adenovirus vector systems can become produced and used to treat cancer and auto-immune diseases.

The invention provides a system to produce and deliver intracellularly recombinant proteinaceous substances comprising TK-apoptin or functional equivalent or functional fragments there of, or recombinant proteinaceous substances with TK-apoptin-like activity. The invention further provides for the addition of further optional modular peptides. This can include an epitope tag, allowing easy detection and immuno-precipitation without direct steric hindrance of associations of TK-apoptin with cellular proteins.

The invention provides or describes all steps needed for the production of recombinant apoptosis inducing agent TK-apoptin, or derivatives of TK-apoptin that have a similar tumor specificity. The recombinant protein can be produced in *E. coli*, insect cells by means of a baculovirus-based vector, or in yeast strains (such as *Pichia pastoris*). The invention provides evidence that the TK-apoptin or TK-apoptin-like proteinaceous substance does not need to be folded properly in the producer cell, enabling the production of recombinant TK-apoptin or TK-apoptin-like proteinaceous substances in transgenic plant cells, for mass production.

The invention proposes a modified metal affinity tag for optimal purification of the recombinant protein. By using a double His-tag next to the transduction domain, a significantly better binding to Nickel beads is achieved, resulting in the possibility to wash the recombinant TK-apoptin or TK-apoptin-like proteinaceous substances under very stringent conditions resulting in an optimal purification of TK-apoptin or TK-apoptin-like proteinaceous substances.

The invention describes the use of a transduction domain fused to recombinant TK-apoptin protein. This domain allows the recombinant protein to pass through the cellular membrane. This domain can consist of a transduction domain derived from HIV TAT, or of any other known transduction domain.

The invention also provides compositions for use in a therapy for cancer, autoimmune diseases or related diseases, which is based on TK-apoptin or apoptin-like proteinaceous substances or virus vector systems, which contain the gene expressing TK-apoptin or TK-apoptin-like proteins.

The invention also provides compositions to remove aberrant cells in their first stages of transformation and pre-malignant lesions. Especially, tumors resistant for chemotherapy.

The invention is further explained by the use of the following illustrative examples.

### EXAMPLES

### Construction of the TK-Apoptin Fusion Cassette.

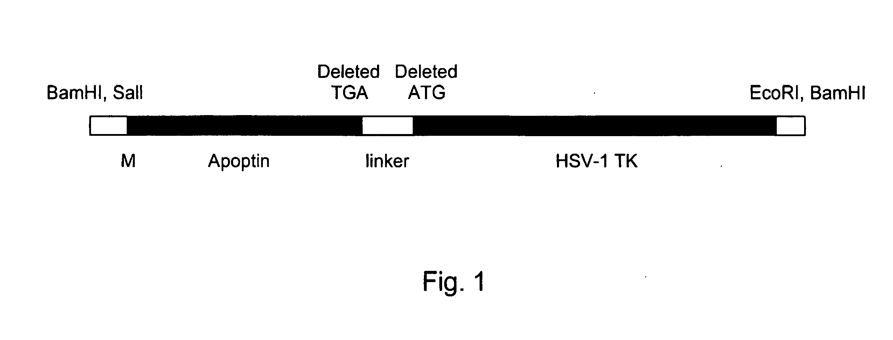

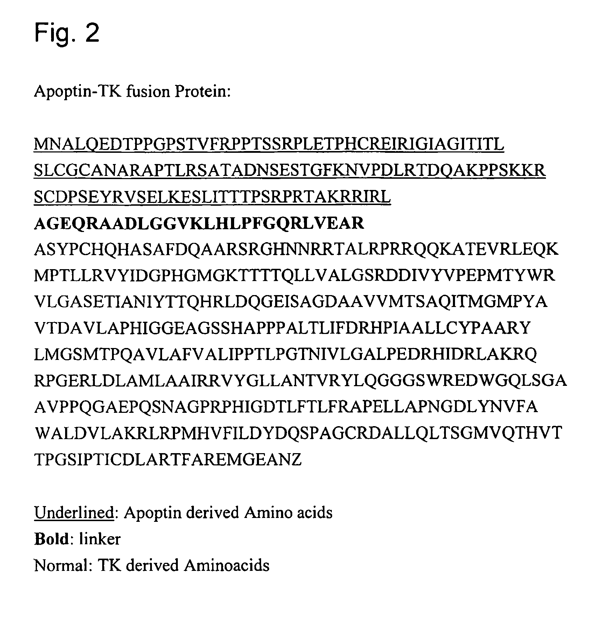

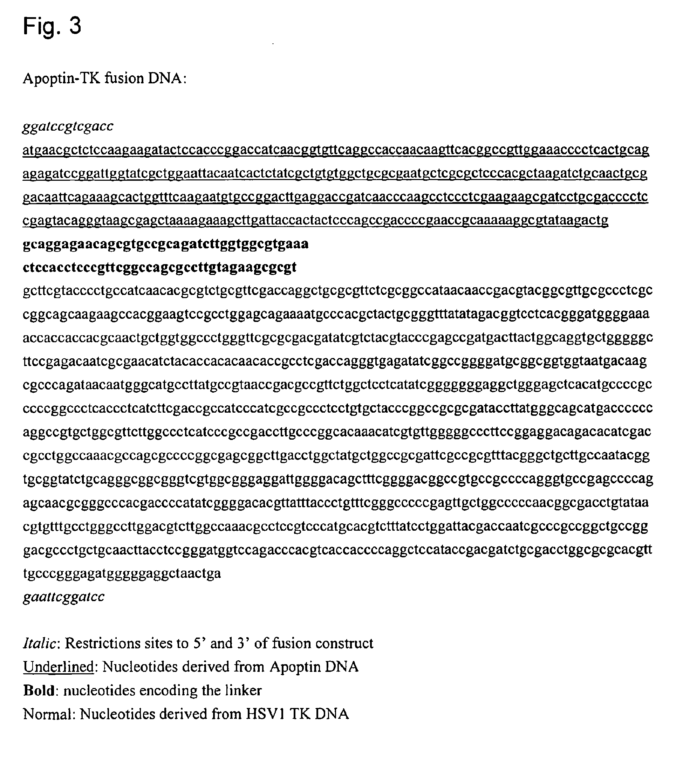

A fusion protein was constructed so that the Apoptin protein was fused through a flexible linker to the N-terminus of the Thymidine Kinase protein. FIG. 1 shows a schematic representation of this construct. FIGs. 2 and 3 show the amino acid sequence as well as the DNA sequence of the TK-Apoptin-fusion product, respectively.

This construct has several features besides the presence of Apoptin and HSV TK encoding regions. Upstream of the initiator ATG of the fusion protein a *BamH*I restriction site was constructed to enable cloning into various expression vectors.

To expedite the cloning of this construct in a protein transduction vector, a unique *Sal*I site was inserted that allows in frame cloning in the pMV TAT vector as described in the patent application recently filed by Noteborn et al. in 2000, which is entitled "A delivery method for the tumor specific apoptosis inducing activity of apoptin, EP 1186665. Such a cloning would result in the addition of a transduction domain N-terminally of Apoptin, and the subsequent possibility of production and purification of recombinant Apoptin-TK fusion protein.

To minimize the risk of steric interference of Apoptin with the TK protein, an oligonucleotide encoding for a 26 amino-acid linker was inserted between the Apoptin encoding ORF and the TK encoding ORF. This linker was based on a paper in which it was shown that a GFP-TK fusion with this linker is functional (Paquin et al., 2001). This linker will also reduce the possibility of negative interference of the TK protein with Apoptin function. The sequence of the linker is given in FIG. 2.

The start methionine ofTK was replaced through the conservative substitution of leucine to minimize the risk of internal initiation at the fusion mRNA, and thus production of TK without the Apoptin moiety. To ensure the production of a full-length TK-apoptin fusion protein, the stopcodon of apoptin was deleted.

Directly after the stop codon of the TK encoding ORF an *EcoR*I and a *BamH*I restriction site was inserted to allow cloning in an expression vector or in pMV TAT.

### Construction of the Transfer Vector AdApt/TK-Apoptin

The AdApt® adenoviral vector contains the cytomegalovirus (CMV) promoter, which has also been optimally adapted to the cell line PER.C6. To examine whether it is possible to produce TK-apoptin by means of an adenovirus vector, we constructed AdApt-Apoptin. To that end, the above described TK-apoptin cassette was cloned into the *BamH*I site of the 6.1-kb transfer vector AdApt, which was obtained from Crucell Holland, bv, Leiden, NL. By sequence analysis and restriction-enzyme digestions the correct orientation of the TK-apoptin gene under the regulation of the CMV was determined. This transfer vector has been named pAdApt/TK-Apoptin. As a negative control, adenovirus transfer vector the plasmids were selected, which contain the TK-apoptin gene in the wrong orientation opposite to the CMV promoter and is named AdApt/TKAAS.

### Construction and characterization of the adenovirus vector AdApt/TK-Apoptin

Next, recombinant adenovirus vectors expressing the TK-apoptin gene under the regulation of the CMV promoter were generated. In addition, also control adenovirus harboring the TK-apoptin gene cassette in the opposite orientation relative to the CMV promoter was made. To that end, PER.C6 cells (Crucell Holland, bv, Leiden, NL) were co-transfected with the adenovirus vector plasmid pAd5AlfII-ITR (E1-, E3+) and with the transfer plasmids pAdApt/TK-Apoptin or pAdApt/TKAAS. After the observation of cytopathogenic effects of the transfected PER.C6, the medium containing the recombinant adenovirus vectors were harvested and plaque-purified (Noteborn and Pietersen, 1998).

The various plaque-purified recombinant adenovirus batches AdApt/TK-Apoptin encoding the TK-Apoptin and control vector AdApt/TKAAS were examined by PCR-analysis for the presence of the TK-Apoptin gene in the 'correct' versus 'wrong' orientation, respectively (Pietersen et al., 1999). All analyzed (in total for each vector type at least 10) recombinant adenovirus batches contained the expected TK-apoptin gene.

Replication-competent adenovirus ("RCA") analysis by means of PCR (Pietersen et al. 1999) revealed that in all analyzed batches no RCA was generated.

Finally, the production of TK-apoptin protein by AdApt-infected human HepG2 cells was examined by means of in-direct immunofluorescence using the monoclonal antibody 111.3 (Noteborn and Pietersen, 1998). The cells were almost all shown to produce TK-apoptin protein and became very soon after infection, apoptotic. This was analyzed by DAPI-staining as well as by means of a TUNEL assay (Pietersen, 1999). This finding is indicative of the fact that the produced TK-apoptin is completely active as an apoptotic inducer. As expected, all cells infected with AdApt/TKAAS did not stain for the monoclonal antibody and did not become apoptotic. In addition, by means of an immunoprecipitation assay and Western-blot analysis using the antibody 111.3, it was shown that the expected TK-Apoptin fusion product of approximately 50 kDa was detectable in the case of HepG2 cells infected with AdApt/TK-apoptin and not visible in HepG2 infected with AdApt/TKAAS or mock-infected.

In conclusion, the production of TK-apoptin by means of the above described recombinant adenovirus vector system, indicates that TK-apoptin can be produced in any adenoviral vector without limiting the adenovirus production.

### In Vitro Treatment of Human Tumor Cells with AdApt/TK-Apoptin and Ganciclovir

Next, we examined whether infection of human tumor cells with AdApt/TK-apoptin and treatment with ganciclovir will result in massive cell death. To that end, human hepatoma HepG2, osteosarcoma U2OS cells, SCC-15 cells, derived from squamous cell carcinoma, and cells from the spontaneously transformed keratinocyte cell line HaCAT (Noteborn and Pietersen, 1998) were used.

First, the various tumor cell lines were transduced with AdLacZ to determine the infective dose per cell line for obtaining 10, 30, or 50% transducibility. Subsequently, the various cell lines were infected with AdApt/TK-apoptin or AdApt/TKAAS. As positive controls, the tumor cells were infected with AdApt-Apoptin (Noteborn and Pietersen, 2000), and as negative controls, cells were also infected with an adenovirus vector expressing TK (van der Eb et al.,1998) or mock-infected. One day after infection, half of the cell cultures was treated with ganciclovir (5 µg per ml; refreshed after every other day).

Six days after infection, the tumor cell cultures were stained with Giemsa as described by Noteborn and Pietersen (1999). All of the tumor cell cultures that were mock-infected or infected with AdTKAAS were rather viable, as well as the tumor cell cultures infected with AdTK expressing HSV thymidine kinase. At most, a slight effect on the cell viability of ganciclovir treatment was seen for the AdTKAAS- or AdTK-infected cells. The cell cultures that were for 50% transduced/infected with AdApt-apoptin or AdApt/TK-apoptin showed a clearly killing effect, which was not seen for cell cultures transduced for 50% with AdTK. These results show that Apoptin (as expected) or TK-Apoptin both harbor a cytotoxic effect in the absence of ganciclovir.

Upon ganciclovir treatment, the amount of cells of the four analyzed tumor cell cultures transduced for 10% with AdTK or AdTK-apoptin were strongly reduced. These data indicate that both TK and TK-apoptin have a cytotoxic effect, that is mainly mediated by a by-stander effect. The HepG2 cells were showing the best by-stander effect upon TK-apoptin treatment. At a transduction rate of 10%, almost all HepG2 cells were dead.

### Treatment of Normal Rat Hepatocytes

The most important drawback of the adenovirus-TK/ganciclovir system is the dramatic effect on the viability of normal hepatocytes as, for example, reported by van der Eb et al., 1998. Therefore, we tested the effect of AdApt/TK-apoptin in comparison with AdTK on rat hepatocytes. To that end, cultures with freshly isolated rat hepatocytes were infected with AdTk or AdApt/TK-apoptin and treated with ganciclovir. By means of in-direct immunofluorescence assays, which were carried out in parallel it was determined that about 5-10% of the normal rat liver cells were producing TK or TK-apoptin, respectively. As negative control, the rat hepatocytes were mock-infected.

One day after infection, ganciclovir was added as described above for the human tumor cell lines. Four days after infection, the cells were stained with Giemsa. The amount of hepatocytes infected with AdTK or AdApt/TK-apoptin was similar to those that were mock-infected. The hepatocyte cultures that were infected with AdTK and treated with ganciclovir, however, were strongly reduced in the amount of hepatocytes. In contrast, hepatocytes infected with AdApt/TK-Apoptin and treated with ganciclovir did only show a slight reduction of the amount of hepatocytes.

These data clearly show that TK-apoptin can induce cell death in tumor cells, which are containing TK-apoptin product and also in tumor cells that were not transduced, by means of a by-stander effect. More importantly, normal (rat) hepatocytes, which are killed by Ad-TK/ganciclovir treatment are viable upon AdApt/TK-apoptin and ganciclovir treatment. The apoptin tumor-specific apoptosis characteristics seem to hold also in the setting of TK-apoptin fusion protein, delivered by an adenoviral vector.

Most likely, Apoptin harbors an activity that can sequester the TK-Apoptin fusion products in cytoplasmic structures of normal human cells, which also has been described for MBP-apoptin fusion product in a previous US patent application by Noteborn et al. (entitled "A delivery method for the tumor-specific apoptosis inducing activity of apoptin", co-owned US provisional no. 60/236,117, now US Patent Appln. Serial no. 09/949,780).

### Treatment of Human RA-derived fibroblast-like Synoviocytes and Normal Fibroblast Synoviocytes with AdApt/TK-apoptin.

Next, we tested the activity of AdApt/TK-apoptin in RA-derived fibroblast synoviocytes versus ones derived from healthy persons (material was obtained from the Rheumatology Department, Leiden University Medical Center, Leiden, NL). As positive control, cells were infected with AdApt-apoptin or AdTk and as negative control cells were mock-infected. In parallel immunofluorescence analysis, showed that about 5-10% of the cells were infected. Harvesting of the cells and Giemsa staining was carried out as described above.

Upon ganciclovir treatment, both normal as well as RA-derived cells treated with AdTK underwent significantly cell death, which was only the case for RA-derived cells treated with AdApt/TK-apoptin and not for the cells derived from healthy persons. All the other cell cultures did not reveal a visible reduction of cell amount.

These data imply that TK-apoptin also has a specific cell-killing activity in RA-related cells, which is at least dramatically reduced in normal cells derived from healthy individuals.

### Production of recombinant TK-apoptin protein

The production and purification of recombinant MBP-TK-apoptin protein has been carried out in an *E. coli* system as has been described for MBP-apoptin by Noteborn et al. (EP 1186665).

### Tumor-specific Induction of Apoptosis

To assay the ability of MBP-TK-apoptin protein to specifically induce apoptosis in tumor cells, a micro-injection system was set up. Human osteosarcoma Saos-2 cells and normal human VH10 fibroblasts were cultured on glass cover-slips. The cells were micro-injected in the cytoplasm with MBP-TK-apoptin protein or as positive control MBP-apoptin or MBP using an Eppendorf micro-injector with the injection pressure of 0.5 psi. The cells were co-injected with dextran-rhodamine to be able to later identify the micro-injected cells.

The cells were fixed and analyzed by using the methods described by Noteborn et al. (EP 1186665).

The results show that both MBP-apoptin and MBP-TK-apoptin were able to induce rapid apoptosis in human tumor cells (within 3-6 hours), but did not induce apoptosis in normal human cells. The MBP control protein did not induce apoptosis in any of the cell preparations tested under these conditions. Remarkably, the apoptin fusion proteins were present in the nucleus of tumor cells, but sequestered in cytoplasmic structures within the VH10 cells.

The following observations may help to further explain the tumor-specific activity of MBP-apoptin and MBP-TK-apoptin. Twenty-four hours after microinjection, almost all tumor cells (apoptotic at this time point) contained MBP-apoptin or MBP-TK-apoptin. The tumor cells (viable) were positive for MBP upon microinjection of MBP protein. The VH10 cells, however, microinjected with MBP-apoptin or MBP-TK-apoptin were negative when stained for MBP or apoptin, but still contained dextran-rhodamine. In contrast, VH10 cells micro-injected with MBP protein still contained MBP and the MBP-signal was not declined in comparison with MBP-microinjected cells that were harvested after 1 or 3 hours.

These data show that the recombinant proteinaceous substance TK-apoptin can induce apoptosis in tumor cells, and not in normal non-transformed cells. This implies that TK does not interfere with the tumor-specific induction of apoptosis by apoptin and is inactivated by proteolytic degradation or shielding.

To explore this issue further, we decided to use a biochemical approach. We had already noticed that transfected apoptin could be readily immunoprecipitated with stringent RIPA buffer, but not at all in mild buffer (Noteborn and Rohn, unpublished observations), suggesting that normal-specific epitope shielding was the more likely possibility. With this in mind, we decided to evaluate epitope shielding by immunoprecipitation compared with the amount of protein actually present in the lysate without immunoprecipitation.

First, we seeded VH10 cells and Saos-2 cells and the next day, microinjected MBP-apoptin protein or MBP-TK-apoptin protein into the cytoplasm of 500 cells per dish. At 1, 6 and 24 hours following microinjection, cell lysates were made with mild lysis buffer. The supernatant of each clarified lysate was split into two equal aliquots, resuspended in sample buffer, resolved by SDS-PAGE, and subjected to Western blot analysis. The recombinant MBP-apoptin and MBP-TK-apoptin in one aliquot was detected by antibodies against the C-terminal region of apoptin (VP3-C) and in another by antibodies directed against MBP. Non-injected cell lysate was analyzed in parallel for negative control.

Whereas MBP-apoptin or MBP-TK-apoptin could be readily immunoprecipitated from injected tumor cells at both time points, as assessed by VP3-C staining on the Western blot. However, in normal cells MBP-TK-apoptin or MBP-apoptin was not pulled down efficiently even at one hour after microinjection, and after 6 hours it could not be immunoprecipitated at all. This phenomenon was also seen when the Western blot was reprobed with an antibody against MBP, although the shielding was not as marked here.

This result suggests that the entire molecule might not be readily available under native conditions, perhaps due to its aggregation capability.

Next, VH10 cells were microinjected with MBP-apoptin protein or MBP-TK-apoptin protein (of each 500 cells per dish), and lysed at 3h (early time) and 24h (late time) after microinjection with mild lysis buffer versus the strongly denaturing RIPA lysis buffer. Each lysate supernatant of identical cells was equally divided into two aliquots. One aliquot was immunoprecipitated with VP3C antibodies. Another aliquot was directly added into sample buffer for Western blotting without immunoprecipitation. Meanwhile, non-injected cell lysates were made with both lysis buffer as controls. All samples were separated on parallel 12.5% SDS-PAGE and electroblotted. Blots were incubated with the antibody MBP-probe and probed with ProtA-HRP for immunoprecipiteated samples and GαR-HRP for non-immunoprecipitated samples. Without immunoprecipitation, MBP-apoptin or MBP-TK-apoptin from the lysate made with either mild lysis buffer or RIPA lysis buffer was equally detected by antibodies directed against MBP at both early (3h) and late (24h) time points. However, the apoptin-specific antibody did not strongly immunoprecipitate MBP-apoptin or MBP-TK-apoptin from the lysate made with mild lysis buffer, allowing only very weak detection of MBP-apoptin or MBP-TK-apoptin at the early time point (3h) and a complete failure to detect at the late time point (24h). In contrast, VP3C antibodies efficiently immunoprecipitated MBP-apoptin or MBP-TK-apoptin from the lysate made with RIPA lysis buffer at both early (3h) and late (24h) time points.

These results strongly suggest that the negative detection of MBP-apoptin in normal cells is due to the epitope shielding on the Apoptin moiety of the protein, a shielding which grows more intense with time post-injection.

To determine the long-term fate of MBP-apoptin and MBP-TK-apoptin in normal human cells, we again microinjected VH10 cells with MBP-apoptin protein or MBP-TK-apoptin protein, MBP protein or Rho-Dex alone as described above. Cells were lysed in the stringent RIPA-buffer at 1,2,3,4 and 5 days after microinjection and analysed by Western-blotting using antibodies directed against apoptin or MBP as mentioned above. Both MBP and Rho-Dex staining was still prevalent after 5 days, whereas MBP-apoptin or MBP-TK-apoptin disappeared already completely after 2 days.

These data clearly show that MBP-TK-apoptin and MBP-apoptin, due to normal-specific features, become degraded.

The fact that apoptin enables degradation of the fusion protein of which it is part of, is further given by the following microinjection and fluorescence-microscope experiments. MBP-apoptin was labeled with fluorescein (kind gift of Rutger Leliveld, University of Leiden, The Netherlands). Microinjection of Saos-2 cells with the fluorescein-labeled MBP-apoptin protein resulted in induction of apoptosis in a similar rate as was described for MBP-apoptin, which was not labeled with fluorescein. Furthermore, fluorescein-labeled apoptin was detectable in these tumor cells, at least for 2 days after microinjection.

Microinjection of fluorescein-labeled MBP-apoptin in VH 10 cells resulted in the sequestering of labeled MBP-apoptin in the same cytoplasmic structures as observed for MBP-apoptin. After 24 hours, fluorescence microscope analysis showed that almost all microinjected VH10 cells did not contain detectable amounts of fluorescein-labeled MBP-apoptin.

These data clearly show that in normal cells apoptin(fusion protein) will become sequestered in cytoplasmic structures, and, subsequently becomes degraded and thus functionally inactivated. These data are also confirmed by observations in our laboratory that the level of apoptin protein in normal splenocytes derived from transgenic-apoptin mice increases dramatically upon addition of proteasomal inhibitors (Pietersen and Noteborn, personal communication).

### MBP-apoptin and MBP-TK-apoptin do not induce apoptosis in other human primary cells.

Because certain human primary cell types are known to be more sensitive than fibroblasts to the effects of standard tumor therapy, such as chemo- or radiation regimens, we decided to exploit the utility of the microinjection technique to circumvent problems of poor transfection efficiency and determine whether apoptin was toxic in these more unusual cells. To this end, we microinjected human primary hepatocytes and human primary mesenchymal stem cells with MBP-apoptin protein, MBP-TK-apoptin protein and MBP protein. As a positive control to confirm that the cells were competent to undergo apoptosis, we also microinjected a plasmid encoding the death-effector FADD (Chinnaiyan and Dixit, 1996) into the nucleus of these cells at one time point.

Both cell types, although exquisitely sensitive to FADD-induced apoptosis, remained resistant to the effects of MBP-apoptin and MBP-TK-apoptin. Instead, MBP-apoptin and MBP-TK-apoptin remained harmlessly in the cytoplasm and formed cytoplasmic aggregation structures as observed for VH10 cells (see above). Twenty four hours after microinjection with MBP-apoptin or MBP-TK-apoptin, almost all human hepatocytes and mesenchymal stem cells were negative when stained for MBP or apoptin, but still contained dextran-rhodamine as has been seen also for VH10 cells. In contrast, human hepatocytes and mesenchymal stem cells microinjected with MBP protein still contained clearly detectable amounts of MBP.

Next, we wanted to evaluate one of the most sensitive of all cell types, CD34+ bone marrow stem cells. Because these cells are grown in suspension, we first affixed them to the microinjection plate using a coating of Wheat Germ Agglutinin, because this cell type is known to possess many receptors for this lectin. Cells were cultured overnight in medium tailored to retain their primitive characteristics. We were unable to microinject the limited cytoplasmatic area of this tiny cell type with the smallest commercially available needles, so we used a plasmid encoding wild-type apoptin or TK-apoptin to perform the test, compared with a plasmid encoding the non-apoptotic desmin gene as negative control, and FADD as a positive control. Although FADD was highly toxic in this experiment, apoptin or TK-apoptin did not induce above-background levels of apoptosis.

Thus gene or protein therapy with (TK)-apoptin is likely to have a larger therapeutic window due to low side effects in normal tissue, even especially sensitive ones.

### Prevention of tumor cell growth in vitro by TAT-TK-Apoptin

The production of MBP-TK-apoptin-TAT protein has been carried out in the same vectors, production systems and purification methods as has been described for MBP-apoptin(vp3)-TAT protein (EP 1186665).

To examine whether MBP-TK-apoptin-TAT can cause specifically apoptosis in human tumor cells, Saos-2 and U2OS (tumor) and VH10 (normal) cells were split 1:10 and cultured for 1 week in the presence of 1 microgram/ml MBP-TK-apoptin-TAT protein or MBP-apoptin-TAT, or in normal medium. Half of the cultures were treated with ganciclovir as described above from day 2 onwards. Subsequently, the cells were fixed with methanol-acetic acid and stained with Coomassie Blue. Although the cells in the control medium had all grown to confluency, the Saos-2 and U2OS cells treated with MBP-TK-apoptin-TAT or MBP-apoptin-TAT had significantly undergone apoptosis. The cells treated MBP-TK-apoptin-TAT protein and ganciclovir completely disappeared from the dish. The VH10 cells treated with MBP-apoptin-TAT or MBP-TK-apoptin-TAT in the vicinity of ganciclovir or not had all also grown to the same density as control treated VH10 cells, showing that MBP-TK-apoptin-TAT does not affect the growth of non-transformed cells.

### REFERENCES

Aghi, M., Hochberg, and Breakefield, X.O. (2000). Prodrug activation enzymes in cancer gene therapy. Journal Gene Medicine 2, 148-164.
Aupperle, K.R., Boyle, D.L., Hendrix, M., Seftor, E.A., Zvaifler, N.J., Barbosa, M., and Firestein, G.S. Regulation of synoviocyte proliferation, apoptosis, and invasion by the p53 tumor-suppressor gene. (1998). American J. Pathology 152, 1091-1098.
Bellamy, C.O.C., Malcomson, R.D.G., Harrison, D.J., and Wyllie, H. 1995. Cell death and disease: The biology and regulation of apoptosis. Seminars in Cancer Biology 6, 3-12.
Bucala, R., Ritchlin, C., Winchester, R., Cerami, A. Constitutive production of inflammatory and mitogenic cytokines by rheumatoid synovial fibroblasts. (1991). J. Experimental Medicine 173, 569-574.
Chinnaiyan, A. M. and Dixit, V. Induction of apoptosis by tumor suppressor genes and oncogenes. Seminars in Cancer Biology, 6: 17-25, 1996. Danen-Van Oorschot, A.A.A.M., Fischer, D.F., Grimbergen, J.M., Klein, B., Zhuang, S.-M., Falkenburg, J.H.F., Backendorf, C., Quax, P.H.A., Van der Eb, J.A., and Noteborn, M.H.M. (1997). Apoptin induces apoptosis in human transformed and malignant cells but not in normal cells. Proceedings National Academy Sciences, USA: 94, 5843-5847.
Danen-Van Oorschot et al. (1997a). BAG-1 inhibits p53-induced but not apoptin-induced apoptosis. Apoptosis 2, 395-402.
Derossi, D., Chassaing, G., and Prochiantz, A. (1998). Trojan peptides: The penetration system for intracellular delivery. Trends in Cell Biology 8, 84-87.
Duke, R.C., Ocjius, D.M., Young, J, D-E. (1996). Cell suicide in health and disease. Scientific American December 1996, 48-55.
Earnshaw, W.C., 1995. Nuclear changes in apoptosis. Current Opinion in Cell Biology 7, 337-343.
Fels, C., Schafer, C., Huppe, B., Bahn, H., Heidecke, V., Kramm, C.M., Lautenschlager, C., and Rainov, N.G. (2000). Bcl-2 expression in higher-grade human glioma: a clinical and experimental study. Journal of Neurooncology 48, 207-216.
Fawell, S., Seery, J., Daikh, Y., Moore, C., Chen, L., Pepinsky, S., and Barscum, J. Tat-mediated delivery of heterologous proteins into cells. Proceedings National Academic Sciences USA 91, 664-668.
Firestein, G.S. Invasive fibroblast-like synoviocytes in rheumatoid arthritis. (1995). Arthritis and Rheumatism 39, 1781-1790.
Firestein, G.S. Apoptosis in rheumatoid arthritis synovium. (1995a). J. Clinical Investigations 96, 1631-1638.
Firestein, G.S. Rheumatoid arthritis: Etiology and Pathogenesis of Rheumatoid arthritis. (1997). Textbook of Rheumatology, 5th edition. Eds. Kelley, Harris, Ruddy and Sledge. Chapter 54, pp 851-897.
Firestein, G.S., Novel therapeutic strategies involving animals, arthritis, and apoptosis. (1998). Current opinion in Rheumatology 10, 236-241.
Frankel, A.D., and Pabo, C.O. (1988). Cellular uptake of the Tat protein from human immunodeficiency virus Cell 55, 1189-1193.
Gay, S. and Gay, R.E. Cellular basis and oncogene expression of rheumatoid joint destruction. (1989). Rheumatology International 9, 105-113.
Geiler, T., Kriegsmann, J., Keyzer, G.M., Gay, R.E., Gay, S. A new model for rheumatoid arthritis generated by engraftment of rheumatoid synovial tissue and normal human cartilage into SCID mice. (1994). Arthritis Rheumatology 37, 1664-1671.
Green, M., and Loewenstein, P.M. (1988). Autonomous functional domains of chemically synthesized human immunodeficiency virus Tat trans-activator protein. Cell 55, 1179-1188.
Hawiger, J. (1999). Non-invasive intracellular delivery of functional peptides and proteins. Current Opinion in Chemical Biology 3, 89-94.
Hockenberry, D.M. (1994). Bcl-2 in cancer, development and apoptosis. Journal of Cell Science, Supplement 18, 51-55.
Kerr, J.F.R., Winterford, C.M., and Harmon, B.V. (1994). Apoptosis: Its significance in cancer and cancer therapy. Cancer 73, 2013-2026.
Lafyatis et al. "Anchorage-independent growth of synoviocytes from arthritic and normal joints: stimulation by exogenous platelet-derived growth factor and inhibition of by transforming growth factor beta and retinoids", Journal Clinical Investigations 83, 1267-1276 (1998).
Levine, A.J. (1997). p53, the cellular gatekeeper for growth and division. Cell 88, 323-331.
Lindgren, M., Haelbrink, M., Prochiantz, A., and Langel, U. (2000). Cell penetrating peptides, Trends in Pharmacological Sciences 21, 99-103.
McDonell T.J., Meyn, R.E., Robertson, L.E. (1995). Implications of apoptotic cell death regulation in cancer therapy. Seminars in Cancer Biology 6, 53-60.
Noteborn, M.H.M. (1996). PCT application WO 96/41191. Apoptin induces apoptosis in human transformed and malignant cells but not in normal cells as essential characteristic for the development of an anti-tumor therapy.
Noteborn, M.H.M., and De Boer, G.F. (1996) U.S. Patent Appln serial no. 030, 335.
Noteborn, M.H.M. and Pietersen, A.M. (2000). Reuma. PCT/NL00/00013.
Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.
Noteborn, M.H.M., and Pietersen, A. (1998). A gene delivery vehicle expressing the apoptosis-inducing proteins VP2 and/or apoptin. PCT Application no. PCT/NL98/00213
Noteborn, M.H.M., Todd, D., Verschueren, C.A.J., De Gauw,H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.
Noteborn, M.H.M., Verschueren, C.A.J., Koch, G., and Van der Eb, A.J. (1998). Simultaneous expression of recombinant baculovirus-encoded chicken anemia virus (CAV) proteins VP1 and VP2 is required for formation of the CAV-specific neutralizing epitope. Journal General Virology, 79, 3073-3077.
Noteborn, M.H.M., and Zhang, Y. (1998). Methods and means for determining the transforming capability of agents, for determining the predisposition of cells to become transformed and prophylactic treatment of cancer using apoptin-like activity. PCT Application no. WO 99/08108.
Noteborn et al. (1998a). Chicken anemia virus: Induction of apoptosis by a single protein of a single-stranded DNA virus. Seminars in Virology 8, 497-504.
Paquin A, Jaalouk DE, Galipeau J. (2001). Retrovector encoding a green fluorescent protein-herpes simplex virus thymidine kinase fusion protein serves as a versatile suicide/reporter for cell and gene therapy applications. Human Gene Therapy 12,13-23.
Paulovich, A.G., Toczyski, D., Hartwell, H. (1997). When checkpoints fail. Cell 88, 315-321.
Pietersen, A.M., Van der Eb, M.M., Rademaker, H.J., Van den Wollenberg, D.J.M., Rabelink, M.J.W.E., Kuppen, P.J.K., Van Dierendonck, J.H., Van Ormondt, H., Masman, D., Van de Velde, C.J.H., van der Eb, Hoeben, R.C., and Noteborn, M.H.M. (1999). Specific tumor-cell killing with adenovirus vectors containing the apoptin gene. Gene Therapy 6, 882-892.
Remmers, E.F., Lafyatis, R., Kumkumian, G.K., Case, J.P., Roberts, A.B., Sporn, M.B., and Wilder, R.L. Cytokines and growth regulation of synoviocytes from patients with rheumatoid arthritis and rats with streptococcal cell wall arthritis. (1990). Growth Factors 2, 179-188.
Sachs, L. and Lotem, J. (1993). Control of programmed cell death in normal and leukemia cells: New implications for therapy. Blood 82, 15-21.
Steller, H. (1995). Mechanisms and genes of cellular suicide. Science 267, 1445-1449.
Schwarze, S.R., Ho, A., Vocero-Akbani, A., and Dowdy, S. (2000)."In vivo protein transduction: delivery of a biologically active protein into the mouse", Science 285, 1569-1572.
Teodoro, J.G. and Branton, P.E. (1997). Regulation of apoptosis by viral gene products. Journal of Virology 71, 1739-1746.
van der Eb et al. (1998). Severe hepatic dysfunction after adenovirus-mediated transfer of the Herpes Simplex thymidine-knase gene and ganciclovir administration. Gene Therapy 5, 451-458.
Vives, E., Brodin, P., and Lebbleu, B. (1997). A truncated HIV-I Tat protein basic domain rapidly translocates through the plasma membrane and accumulates in the cell nucleus. Journal of Biological Chemistry 272, 16010-16017.
White, E. (1996). Life, death, and the pursuit of apoptosis. Genes and development 10, 1-15.
Wyllie, A.H. (1995). The genetic regulation of apoptosis. Current Opinion in Genetics and Development 5, 97-104.
Wyllie, A.H., Kerr, J.F.R., Currie, A.R. (1980). Cell death: The significance of apoptosis. International Review of Cytology 68, 251-306.
Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells in vitro. Leukemia 9 Sl, 118-120.
Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.

## Claims

1. A fusion protein comprising a polypeptide providing cytotoxicity, said polypeptide being fused to a moiety rendering the fusion protein functionally available in aberrant cells and not functionally available in non-aberrant cells, wherein said moiety is apoptin and wherein said polypeptide provides enzymatic activity that converts a prodrug into a drug.

2. The fusion protein of claim 1, wherein said polypeptide is TK.

3. The fusion protein of claim 1, wherein said fusion protein is conjugated to a targeting molecule.

4. The fusion protein of claim 3 wherein the targeting molecule is selected from the group consisting of liposomes, folic acid and folic acid derivatives, vitamin B-12 and vitamin B-12 derivatives, an antibody or antibody fragment, and a ligand for a receptor.

5. An in vitro method of inducing cell death in aberrant cells, said method comprising: administering a fusion protein having a moiety rendering the fusion protein functionally available in aberrant cells and not functionally available in non-aberrant cells, wherein said moiety is apoptin and wherein said polypeptide provides enzymatic activity that converts a prodrug into a drug.

6. A gene delivery vehicle encoding a fusion protein comprising a moiety rendering the fusion protein functionally available in aberrant cells and not functionally available in non-aberrant cells, wherein said moiety is apoptin and wherein said polypeptide provides enzymatic activity that converts a prodrug into a drug.

7. The gene delivery vehicle of claim 6, wherein the gene encoding the fusion protein encodes a targeting polypeptide.

8. The gene delivery vehicle of claim 7, wherein the targeting polypeptide comprises a transduction domain.

9. The gene delivery vehicle of claim 8, wherein said transduction domain comprises TAT.

10. The gene delivery vehicle of claim 7, wherein the targeting polypeptide comprises a member of a specific binding pair.

11. The gene delivery vehicle of claim 7, wherein the targeting polypeptide comprises a scFv.

12. The gene delivery vehicle of claim 6, wherein the gene encoding the fusion protein encodes two proteins.

13. An in vitro method for providing aberrant cells predominantly over normal cells with a desired fusion protein comprising administering a fusion protein having a moiety rendering the fusion protein functionally available in aberrant cells and not functionally available in non-aberrant cells, wherein said moiety is apoptin and wherein said polypeptide provides enzymatic activity that converts a prodrug into a drug.

14. Use of a fusion protein according to any one of claim 1 to 4 or a gene delivery vehicle according to any one of claims 6 to 12 in the preparation of a medicament for the treatment of cancer and/or auto-immune disease.

## Patentansprüche

1. Fusionsprotein, umfassend ein Polypeptid, das Cytotoxizität vorsieht, wobei das Polypeptid an eine Einheit fusioniert ist, welche das Fusionsprotein in anomalen Zellen funktionell verfügbar macht und in nicht-anomalen Zellen nicht funktionell verfügbar macht, wobei die Einheit Apoptin ist und wobei das Polypeptid eine enzymatische Aktivität bereitstellt, die einen Proarzneistoff in einen Arzneistoff umwandelt.

2. Fusionsprotein von Anspruch 1, wobei es sich bei dem Polypeptid um TK handelt.

3. Fusionsprotein von Anspruch 1, wobei das Fusionsprotein an ein Targeting-Molekül konjugiert ist.

4. Fusionsprotein von Anspruch 3, wobei das Targeting-Molekül aus der Gruppe gewählt ist, die aus Liposomen, Folsäure und Folsäurederivaten, Vitamin-B12 und Vitamin-B12-Derivaten, einem Antikörper oder einem Antikörperfragment und einem Liganden für einen Rezeptor besteht.

5. In-vitro-Verfahren zum Induzieren des Zelltods in anomalen Zellen, wobei das Verfahren folgendes umfasst:
Verabreichen eines Fusionsproteins, das eine Einheit aufweist, welche das Fusionsprotein in anomalen Zellen funktionell verfügbar macht und in nicht-anomalen Zellen nicht funktionell verfügbar macht, wobei die Einheit Apoptin ist und wobei das Polypeptid eine enzymatische Aktivität bereitstellt, die einen Proarzneistoff in einen Arzneistoff umwandelt.

6. Gen-Zuführungsvehikel, das ein Fusionsprotein codiert, das eine Einheit umfasst, welche das Fusionsprotein in anomalen Zellen funktionell verfügbar macht und in nicht-anomalen Zellen nicht funktionell verfügbar macht, wobei die Einheit Apoptin ist und wobei das Polypeptid eine enzymatische Aktivität bereitstellt, die einen Proarzneistoff in einen Arzneistoff umwandelt.

7. Gen-Zuführungsvehikel von Anspruch 6, wobei das Gen, welches das Fusionsprotein codiert, ein Targeting-Polypeptid codiert.

8. Gen-Zuführungsvehikel von Anspruch 7, wobei das Targeting-Polypeptid eine Transductin-Domäne umfasst.

9. Gen-Zuführungsvehikel von Anspruch 8, wobei die Transductin-Domäne TAT umfasst.

10. Gen-Zuführungsvehikel von Anspruch 7, wobei das Targeting-Polypeptid ein Mitglied eines spezifischen Bindungspaars umfasst.

11. Gen-Zuführungsvehikel von Anspruch 7, wobei das Targeting-Polypeptid einen scFv umfasst.

12. Gen-Zuführungsvehikel von Anspruch 6, wobei das Gen, welches das Fusionsprotein codiert, zwei Proteine codiert.

13. In-vitro-Verfahren zum Versehen anomaler Zellen, in vorherrschender Weise gegenüber normalen Zellen, mit einem gewünschten Fusionsprotein, umfassend das Verabreichen eines Fusionsproteins, das eine Einheit aufweist, welche das Fusionsprotein in anomalen Zellen funktionell verfügbar macht und in nicht-anomalen Zellen nicht funktionell verfügbar macht, wobei die Einheit Apoptin ist und wobei das Polypeptid eine enzymatische Aktivität bereitstellt, die einen Proarzneistoff in einen Arzneistoff umwandelt.

14. Verwendung eines Fusionsproteins gemäß mindestens einem von Anspruch 1 bis 4 oder eines Gen-Zuführungsvehikels gemäß mindestens einem der Ansprüche 6 bis 12 bei der Herstellung eines Medikaments zur Behandlung von Krebs und/oder Autoimmunkrankheit.

## Revendications

1. Protéine de fusion comprenant un polypeptide conférant une cytotoxicité, ledit polypeptide étant fusionné avec une fraction rendant la protéine de fusion fonctionnellement disponible dans des cellules anormales et non fonctionnellement disponible dans des cellules non anormales, dans laquelle ladite fraction est l'apoptine et dans laquelle ledit polypeptide confère une activité enzymatique qui convertit un promédicament en médicament.

2. Protéine de fusion selon la revendication 1, dans laquelle ledit polypeptide est TK.

3. Protéine de fusion selon la revendication 1, dans laquelle ladite protéine de fusion est conjuguée à une molécule de ciblage.

4. Protéine de fusion selon la revendication 3, dans laquelle la molécule de ciblage est choisie dans le groupe constitué par les liposomes, l'acide folique et les dérivés de l'acide folique, la vitamine B-12 et les dérivés de la vitamine B-12, un anticorps ou un fragment d'anticorps, et un ligand d'un récepteur.

5. Procédé d'induction de mort cellulaire *in vitro* sur des cellules anormales, ledit procédé comprenant l'étape consistant à : administrer une protéine de fusion comprenant une fraction rendant la protéine de fusion fonctionnellement disponible dans les cellules anormales et non fonctionnellement disponible dans les cellules non anormales, dans lequel ladite fraction est l'apoptine et dans lequel ledit polypeptide confère une activité enzymatique qui convertit un promédicament en médicament.

6. Véhicule de délivrance de gène codant une protéine de fusion comprenant une fraction rendant la protéine de fusion fonctionnellement disponible dans des cellules anormales et non fonctionnellement disponible dans des cellules non anormales, dans lequel ladite fraction est l'apoptine et dans lequel ledit polypeptide confère une activité enzymatique qui convertit un promédicament en médicament.

7. Véhicule de délivrance de gène selon la revendication 6, dans lequel le gène codant la protéine de fusion code un polypeptide de ciblage.

8. Véhicule de délivrance de gène selon la revendication 7, dans lequel le polypeptide de ciblage comprend un domaine de transduction.

9. Véhicule de délivrance de gène selon la revendication 8, dans lequel ledit domaine de transduction comprend TAT.

10. Véhicule de délivrance de gène selon la revendication 7, dans lequel le polypeptide de ciblage comprend un membre d'un couple de liaison spécifique.

11. Véhicule de délivrance de gène selon la revendication 7, dans lequel le polypeptide de ciblage comprend un fragment scFv.

12. Véhicule de délivrance de gène selon la revendication 6, dans lequel le gène codant la protéine de fusion code deux protéines.

13. Procédé *in vitro* permettant de fournir de façon prédominante à des cellules anormales, par rapport à des cellules normales, une protéine de fusion souhaitée, comprenant l'étape consistant à administrer une protéine de fusion comprenant une fraction rendant la protéine de fusion fonctionnellement disponible dans les cellules anormales et non fonctionnellement disponible dans les cellules non anormales, dans lequel ladite fraction est l'apoptine et ledit polypeptide confère une activité enzymatique qui convertit un promédicament en médicament.

14. Utilisation d'une protéine de fusion selon l'une quelconque des revendications 1 à 4 ou d'un véhicule de délivrance de gène selon l'une quelconque des revendications 6 à 12, pour la préparation d'un médicament de traitement du cancer et/ou des maladies auto-immunes.
